# FASCICULE DE BREVET EUROPEEN

(11) **EP 1 476 212 B1**
(45) Date de publication et mention de la délivrance du brevet: **12.04.2006**
(21) Numéro de dépôt: 03715035.6
(22) Date de dépôt: 21.01.2003
(51) Int. Cl.: A61M 5/30, A61M 5/44

(54) **INSTALLATION DESTINEE A LA DELIVRANCE DE CALORIES DANS TOUT OU PARTIE D'UN TISSU CELLULAIRE HUMAIN OU ANIMAL**
VORRICHTUNG ZUR VERABREICHUNG VON KALORIEN AN DAS GESAMTE ODER EINEN TEIL DES ZELLGEWEBES BEIM MENSCH ODER TIER
INSTALLATION FOR DELIVERING CALORIES TO ALL OR PART OF HUMAN OR ANIMAL CELL TISSUE

(30) Priorité: 21.02.2002 FR 0202213
(43) Date de publication de la demande: 17.11.2004
(73) Titulaire: Mehier, Henri, F-69002 Lyon (FR)
(72) Inventeur: Mehier, Henri, F-69002 Lyon (FR)
(74) Mandataire: Vuillermoz, Bruno
(86) Numéro de dépôt international: PCT/FR2003/000192
(87) Numéro de publication internationale: WO 2003/070302

(56) Documents cités:
- EP-A- 1 013 297
- WO-A-00/29055
- US-A- 3 490 696
- US-A- 4 265 600
- US-A- 5 116 313

## Description

L'invention concerne une installation destinée à la délivrance de calories au moyen d'un liquide, dans tout ou partie d'un tissu cellulaire humain ou animal. Plus précisément, elle se rapporte à un appareil permettant d'injecter un liquide caloporteur choisi dans le groupe comprenant l'eau, l'eau oxygénée et l'éthanol dans tout ou partie d'organes, en particulier au niveau de cellules cancéreuses

Une des méthodes de traitement des tumeurs cancéreuses consiste à détruire en tout ou partie le tissu cancéreux par administration ciblée de chaleur ou de froid. Ce principe est connu sous la dénomination "thermoablation" et est actuellement mis en oeuvre notamment dans le traitement des métastases hépatiques.

Plusieurs techniques s'appuyant sur le principe de la thermoablation par la chaleur sont aujourd'hui proposées, telles que laser, radiofréquence avec aiguille, la cryothérapie relevant quant à elle de la thermoablation par le froid. Toutefois, ces techniques présentent un certain nombre d'inconvénients. En particulier, le volume de la tumeur traitée reste limité (en pratique de 4 à 5 cm de diamètre) et le temps d'intervention relativement long, de 20 à 30 minutes pour la radiofréquence et la cryothérapie, d'avantage encore pour le traitement au laser.

Le document WO-00/29055 du Demandeur décrit une technique de thermoablation par la chaleur consistant à injecter dans un tube creux, souple muni de perforations et "cousu" au sein de l'organe à traiter, une substance physiquement active, en pratique de l'eau ou de l'eau oxygénée sous forme pressurisée. La température du liquide pressurisé, au contact du tissu à traiter, diminue et l'eau ou l'eau oxygénée redevient liquide au sein même de la tumeur. Le liquide pressurisé est obtenu et injecté au moyen d'un appareil composé d'une unité de chauffage se présentant sous forme d'une bobine métallique sur laquelle est enroulé un tube en inox, directement connecté au tube microperforé. L'eau chemine et est chauffée dans le tube inox au niveau de cette unité à une température voisine de 400 °C et une pression voisine de 250 bars.

Dans le cadre de sa recherche, le Demandeur a découvert que la délivrance d'eau ou d'eau oxygénée, au niveau d'une tumeur devait avantageusement être effectuée avec de faibles volumes d'eau, injectés sous forme pulsée, tant pour des raisons d'efficacité que pour des raisons de sécurité.

L'utilisation de faibles volumes d'eau, en pratique compris entre 0.2 et 1 ml, permet en effet d'éviter les risques de diffusion de chaleur en dehors de la tumeur et partant, la destruction de tissus sains. Le Demandeur a en outre constaté que l'administration de faibles volumes d'eau ou d'eau oxygénée à intervalles réguliers, en pratique compris entre 0.5 et 1 seconde, permettait de traiter des zones de volumes plus importantes et dans un délai plus court qu'avec les techniques connues de l'art antérieur. En outre le régime pulsé permet d'éviter l'échauffement de la partie du tube non en contact avec la tumeur, c'est à dire de la partie du tube reliant l'extérieur de l'organisme interdisant de fait, la destruction, par la chaleur, de tissus sains.

Comme déjà dit, le liquide destiné à être injecté est une substance physiquement active, en pratique de l'eau, de l'eau oxygénée ou encore de l'éthanol, ces deux derniers présentant l'avantage, outre l'apport de calories, d'être chimiquement agressif vis à vis de la tumeur à traiter.

L'invention concerne donc une installation destinée à la délivrance de calories au moyen d'un liquide, dans tout ou partie d'un tissu cellulaire humain ou animal comprenant :
- une unité de chauffage dudit liquide,
- une unité d'injection de liquide dans l'unité de chauffage,
- un moyen de diffusion de liquide chauffé,
- un moyen d'amenée du liquide chauffé depuis l'unité de chauffage jusqu'au moyen de diffusion.

L'installation se caractérise en ce qu'elle présente des moyens de délivrance du liquide chauffé en régime pulsé au niveau du moyen de diffusion.

En pratique, l'installation présente des moyens de délivrance, en régime pulsé, du liquide chauffé à une pression au moins égale à la pression de vapeur saturante du liquide à injecter.

Dans la suite de la description, l'installation sera illustrée en utilisant l'eau comme liquide à injecter, bien que l'eau oxygénée ou encore l'éthanol puissent être également envisagés.

Pour se faire et selon une première caractéristique de l'invention, l'unité d'injection se présente sous forme d'une chambre contenant le liquide à injecter et dans laquelle un piston est mû en translation sous l'action d'un vérin électrique ou pneumatique, dont le déclenchement, la course, la force et la vitesse de déplacement sont déterminés en fonction du rythme, du volume et de la pression d'injection souhaités du liquide, la chambre communiquant avec l'unité de chauffage par l'intermédiaire d'une première valve.

En d'autres termes, l'unité d'injection constitue le premier élément contribuant à assurer la délivrance de calories en régime pulsé, en injectant dans l'unité de chauffage une quantité déterminée d'eau froide à intervalles réguliers, grâce à la combinaison de l'action d'un vérin dont le déclenchement, la course, la force et la vitesse sont programmés en fonction du rythme, du volume et de la pression d'injection souhaités, avec une valve interdisant le retour du liquide injecté dans la chambre.

Plus précisément, le déclenchement et la course du vérin sont programmés en fonction du nombre d'injections souhaité pour délivrer un volume d'eau déterminé, ce dernier dépendant directement de la taille de la tumeur à traiter. Le Demandeur a ainsi constaté qu'on obtenait une nécrose des tissus satisfaisante en utilisant un volume d'eau pressurisée à 400°C représentant environ 5% du volume de la tumeur à traiter. Le déclenchement et la course du vérin sont alors programmés pour obtenir des volumes de liquide injectés compris entre 0.2 et 1 ml à intervalles réguliers de 0.5 à 1 seconde. En pratique, la programmation de la course du vérin est assurée par le réglage du nombre de tour du moteur actionnant le vérin.

Par ailleurs, la pression à laquelle le liquide est injecté dépend de la vitesse de déplacement et de la force du vérin qui sont également programmés. En pratique, la programmation de la vitesse de déplacement du vérin est assurée par le réglage de la vitesse de rotation du moteur actionnant le vérin.

Or, pour pouvoir injecter de faibles volumes d'eau, il est nécessaire de chauffer le liquide à une température très élevée, pour l'eau ou l'eau oxygénée, par exemple de l'ordre de 400°C et plus. A une telle température et à pression atmosphérique, l'eau ou l'eau oxygénée serait vaporisée au niveau de l'unité de chauffage interdisant ainsi la délivrance efficace, sous forme pulsée, de calories au niveau du système de diffusion. Dès lors, pour résoudre le double problème du maintien de la substance sous forme liquide à une telle température et de son administration sous forme pulsée avec des pulses d'une durée comprise en pratique entre 0,1 et 0,2 seconde, la force et la vitesse sont choisies de telle sorte à ce que le liquide soit injecté dans l'unité de chauffage à une pression supérieure d'au moins 50 bars, avantageusement 100 bars à la pression de vapeur saturante du liquide à injecter. Dans le cas d'injection d'eau, la pression d'injection sera fixée à environ 350 bars.

Comme déjà dit, le vérin est un vérin électrique ou pneumatique. En effet, lorsque le traitement est effectué à proximité d'appareils d'imagerie par résonance magnétique, le vérin doit être amagnétique. Dans ce cas, on utilise avantageusement un vérin pneumatique qui chasse dans l'unité de chauffage de petits volumes d'eau jusqu'à obtenir le volume souhaité.

Selon une autre caractéristique, l'installation comprend un réservoir de stockage de liquide destiné à alimenter la chambre de l'unité d'injection, ledit réservoir de stockage étant fixe ou amovible. L'alimentation en liquide de la chambre est avantageusement effectuée avec des volumes prédéterminés correspondant au volume à injecter dans la tumeur. Dans tous les cas, le réservoir est séparé de la chambre par une seconde valve interdisant le retour du liquide dans la chambre, sous l'action du piston.

Une fois le liquide (par exemple l'eau) froid sous pression, transmis à l'unité de chauffage, celui-ci est chauffé jusqu'à une température de 400°C et plus.

Dans un mode de réalisation avantageux et toujours pour assurer le régime pulsé, l'unité de chauffage se présente sous forme d'une bobine métallique incorporant une résistance électrique ou un échangeur de température, autour de laquelle est entouré un tube inox parcouru par ledit liquide. Le diamètre interne du tube est choisi de telle sorte à interdire le mélange de l'eau chaude avec l'eau froide au moment du pulse. Par ailleurs, la longueur du tube est choisie en combinaison avec le diamètre interne en fonction du volume à injecter.

En pratique, le diamètre interne est compris entre 0,1 et 0,5 mm, avantageusement 0,3 mm, la longueur du tube variant en fonction des dimensions de la bobine et étant comprise, en pratique, entre 1 500 et 5 000 mm. De même, la dimension du diamètre externe du tube inox est comprise entre 1 et 2 mm, avantageusement de l'ordre de 1,5 mm.

Lorsque le traitement est effectué à proximité d'appareils d'imagerie par résonance magnétique, la résistance électrique est remplacée par une source alimentée par un fluide caloporteur, le fluide caloporteur étant chauffé à distance de l'unité de chauffage et véhiculé par un tuyau isolé amagnétique (entre 1 et 2 mètres).

Selon une autre caractéristique, pour résoudre le problème du maintien du liquide à injecter sous forme pressurisée à la température souhaitée jusqu'au moyen de diffusion, l'unité de chauffage est séparée du moyen d'amenée, soit par une soupape tarée à une pression au moins égale à la pression de vapeur saturante du liquidé à injecter, soit par une vanne susceptible de résister à une température élevée et pression élevée, respectivement de l'ordre de 400°C et d'au moins 350 bars ; en pratique de l'ordre de 1 000 bars. Lorsque le liquide à injecter est de l'eau, la soupape est tarée à 250 bars pour 400°C.

Dans un mode de réalisation avantageux, l'installation comprend un circuit de dérivation d'eau froide, dont le point de départ est positionné entre l'unité d'injection et l'unité de chauffage tandis que le point d'arrivée est positionné en aval de la soupape, ledit circuit de dérivation étant connecté au point de départ au moyen d'une électrovanne haute pression programmable.

Ce circuit d'eau froide présente un double avantage. Tout d'abord, il permet, pendant le fonctionnement de l'installation, de purger l'eau chaude qui reste dans le moyen de diffusion, réduisant ainsi l'échauffement en dehors des phases d'injection tout en accélérant le rythme des impulsions. En outre, il constitue un système de sécurité dans l'hypothèse où un volume d'eau chaude trop important aurait été injecté, l'opérateur pouvant injecter alors de l'eau froide dans la tumeur.

En pratique, le circuit d'eau froide se présente sous forme d'un tube inox, dont le diamètre extérieur est compris entre 1,2 et 1,8 mm, tandis que le diamètre intérieur est compris entre 0,1 et 0,5 mm.

Selon une autre caractéristique, l'unité de chauffage est séparée du moyen de diffusion par un moyen d'amenée. Pour réduire au maximum l'espace mort présent à la sortie de l'unité de chauffage et assurer la délivrance de liquide pressurisé sous forme pulsée, le moyen d'amenée du liquide chauffé se présente sous forme d'un tube creux souple apte à subir une pression et une température élevées et dont la longueur est comprise entre 40 et 80 cm tandis que le diamètre extérieur est compris entre 100 et 250 µm et le diamètre interne est compris entre 50 et 150 µm. Pour l'eau, la pression et la température sont respectivement supérieures à 250 bars et 400°C. En pratique, le tube est fabriqué en acier inoxydable de qualité A305, en titane, ou encore en un alliage platine/iridium ou nickel/titane. Il peut être en outre recouvert d'une gaine plastique résistant à la température et assez rigide du type silicone, polyimide, polyuréthane. Le tube présente en outre à l'une de ses extrémités un premier joint conique assurant la jonction avec l'unité de chauffage et un second joint conique assurant la jonction avec le moyen de diffusion.

En d'autres termes, et comme il ressort de ce qui précède, la combinaison des caractéristiques :
- du vérin, permettant l'arrivée d'eau froide en pulse dans l'unité de chauffage,
- du tube inox dans lequel le liquide est chauffé, permettant la propulsion d'eau chaude à température homogène et maximale,
- de la soupape, permettant de maintenir la pression égale à la pression de vapeur saturante du liquide à injecter,
- du moyen d'amenée, permettant de réduire le volume mort au maximum, assure la délivrance d'eau sous forme pressurisée en régime pulsé au niveau du système de diffusion.

Le moyen de diffusion peut être de deux types.

Dans un premier mode de réalisation, le moyen de diffusion se présente sous forme d'un tube similaire à celui décrit dans le document WO-00/29055. Plus précisément, le moyen de diffusion se présente sous forme d'un tube creux dont les parois sont munies en tout ou partie de perforations, en particulier les parois en contact avec le tissu à traiter et dont l'extrémité distale est obturée. En pratique, un tel tube est destiné à être rendu solidaire de l'organe à traiter. De même que s'agissant du moyen d'amenée, il est fabriqué en un matériau du type titane, acier inoxydable, alliage platine/iridium ou nickel/titane, et plus généralement en tout matériau apte à supporter une pression et une température respectivement supérieure à 250 bars et 400°C (pour le cas de l'eau).

Par ailleurs et selon une autre caractéristique, le diamètre extérieur du tube est compris entre 100 et 250 µm, tandis que le diamètre intérieur est compris entre 50 et 150 µm.

Comme déjà dit, il est muni de perforations sensiblement circulaires de diamètre compris entre 30 et 70 µm, avantageusement 50 µm.

Dans un second mode de réalisation, le moyen d'amenée se présente sous forme d'une aiguille rigide dont l'extrémité distale est obturée et dont les parois présentent, sur tout ou partie de la longueur de l'aiguille, en particulier les parois en contact avec le tissu à traiter, des perforations de diamètre compris entre 30 et 70 µm, avantageusement 50 µm. Selon une première caractéristique, le diamètre intérieur de l'aiguille est compris entre 0,05 et 0,2 mm, tandis que le diamètre extérieur est compris entre 0,2 mm et 0,7 mm, la longueur étant comprise entre 100 et 200 mm. Cette aiguille est fabriquée en un matériau du type acier ou inox dur, titane ou encore alliage métallique. La jonction de l'extrémité amont de l'aiguille avec le moyen d'amenée est assurée avantageusement par une soudure ou un joint conique.

L'invention et les avantages qui en découlent ressortiront mieux du mode de réalisation suivant à l'appui des figures annexées.
La figure 1 est une représentation schématique de l'installation.
La figure 2 est une représentation schématique de l'unité d'injection.
La figure 3 est une représentation schématique de l'unité de chauffage.
La figure 4 est une représentation schématique du moyen d'amenée.
La figure 5 est une représentation schématique du moyen de diffusion sous forme d'une aiguille.
La figure 6 est une représentation schématique du moyen de diffusion sous forme d'un tube.

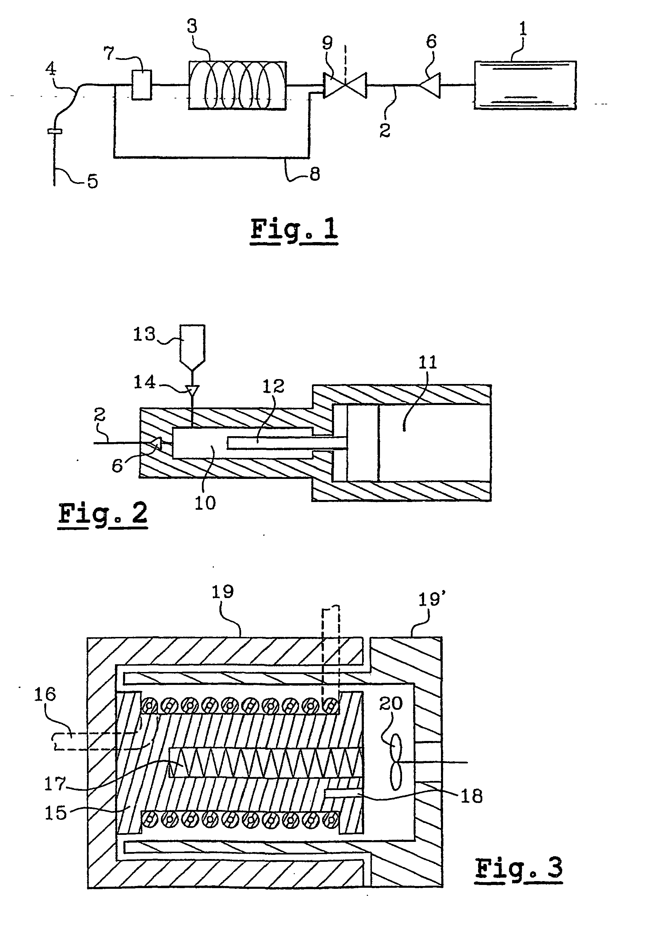

Comme le montre la figure 1, l'installation de l'invention comprend 4 éléments principaux, respectivement une unité d'injection (1) communiquant par le biais d'un tube inox (2) avec une unité de chauffage (3), un moyen d'amenée de liquide chaud ou rallonge (4) et un moyen de diffusion (5). Le diamètre extérieur du tube inox (2) est égal à 1,6 mm tandis que son diamètre interne est égal à 0,25 mm.

Selon une première caractéristique de l'invention, l'unité d'injection est séparée de l'unité de chauffage par une valve (6), l'unité de chauffage (3) étant elle-même séparée de la rallonge (4) par une soupape (7).

Dans le mode de réalisation avantageux, tel que représenté sur la figure 1, l'installation est complétée par un circuit de dérivation d'eau froide (8), dont l'entrée est positionnée entre l'unité d'injection (1) et l'unité de chauffage (3), tandis que la sortie est positionnée en aval de la soupape (7). Par ailleurs, le circuit d'eau froide (8) communique avec le circuit principal d'eau chaude au niveau du point d'entrée par le biais d'une électrovanne programmable (9).

Chaque élément de l'installation va maintenant être revu dans le détail.

S'agissant tout d'abord de l'unité d'injection (1), celle-ci se présente sous forme d'un cylindre muni d'une chambre (10) dans laquelle est mû en translation, sous l'effet d'un vérin (11), un piston (12). Le vérin (11) peut être un vérin électrique ou un vérin pneumatique en fonction de l'environnement dans lequel il est utilisé (nécessité d'un vérin amagnétique ou non). Dans le présent exemple de réalisation, le vérin est un vérin électrique, dont le déclenchement, la course (réglée par le nombre de tour du moteur), la force (réglée par la puissance du moteur) et la vitesse (réglée par la vitesse de rotation du moteur) sont programmables. Dans cet exemple, la force du vérin choisi est égale à 10 Knewtons pour une surface du piston inférieure à 3 cm². Lorsque le liquide utilisé est de l'eau, le vérin est apte à injecter de l'eau froide dans l'unité de chauffage à une pression de 350 bars.

La chambre (10) est en outre connectée à un réservoir de stockage de liquide (13) assurant l'alimentation en liquide de la chambre. Le réservoir de liquide (13) est en outre séparé de la chambre (10) par le biais d'une seconde valve (14).

L'unité de chauffage (3) représentée schématiquement sur la figure 3 se présente sous forme d'une bobine d'aluminium (15), de diamètre égal à 50 mm et de longueur égale à 100 mm, enroulée d'un tube inox (16) de diamètre extérieur égal à 1,6 mm et de diamètre interne égal à 0,25 mm, de longueur comprise entre 1 500 et 5 000 mm constituant le prolongement du tube (2). L'ensemble bobine (15) tube inox (16) incorpore une résistance électrique (17). La bobine (15) est en outre munie d'une sonde (18) régulant la température de la bobine. La bobine est par ailleurs maintenue dans deux tubes concentriques (19, 19') permettant l'isolation thermique et le refroidissement du système en combinaison avec un ventilateur (20).

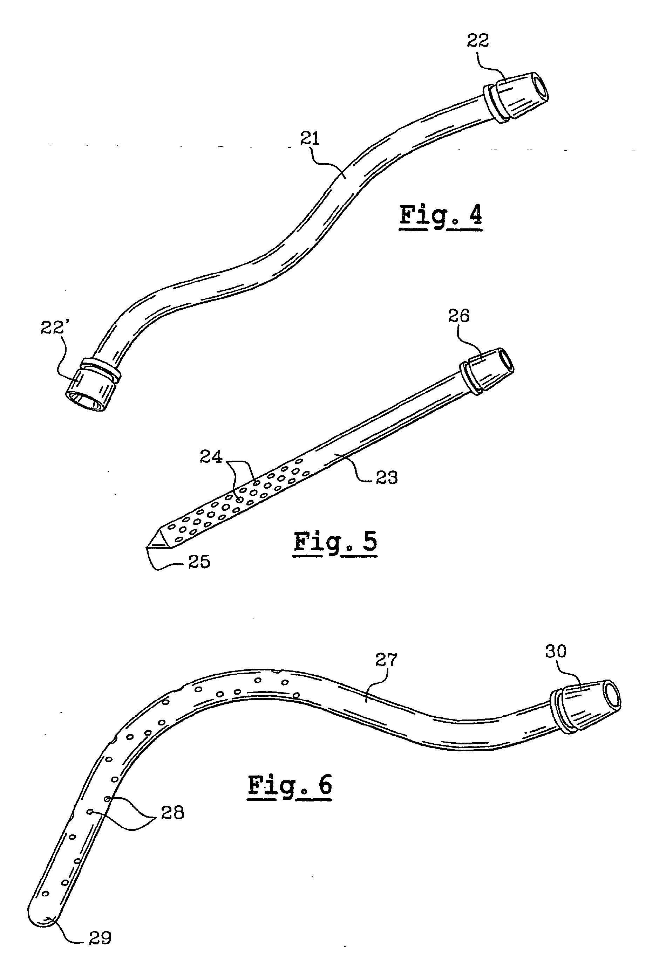

La rallonge (4) telle que représentée sur la figure 4, se présente quant à elle sous forme d'un tube creux (21) de longueur égale à 50 cm et de diamètres interne et externe respectivement égaux à 100 µm et 200 µm. Ce tube est réalisé en acier inoxydable de qualité A305 et recouvert d'une gaine plastique du type silicone. Le moyen d'amenée présente en outre, à son extrémité amont, un premier joint conique (22) permettant le raccordement à l'unité de chauffage et un second joint conique (22') permettant le raccordement au moyen de diffusion.

Comme déjà dit, le moyen d'amenée (4) est séparé de l'unité de chauffage (3) par le biais d'une soupape (7). Selon une caractéristique essentielle, la soupape est tarée à une pression au moins égale à la pression de vapeur saturante du liquide à injecter. Lorsque le liquide injecté est de l'eau, la soupape (7) est tarée à 250 bars 400°C.

Deux types de moyen de diffusion peuvent être utilisés.

Le premier type, représenté en figure 5, correspond à une aiguille en acier (23) de longueur égale à 20 cm environ, obturée à son extrémité distale (25) et munie de perforations (24) sur tout ou partie de sa hauteur, en particulier dans la zone destinée à être en contact avec le tissu à traiter. Le diamètre extérieur de l'aiguille est de 0,5 mm tandis que le diamètre intérieur est égal à 0,2 mm. En outre, les perforations ont un diamètre égal à 50 µm. En pratique, la rallonge (21) est connectée à l'aiguille par soudure (26).

Dans un second mode de réalisation représenté figure 6, le moyen de diffusion (5) se présente sous forme d'un tube (27) destiné à être cousu directement au niveau du tissu. Le tube (27) est muni de perforations (28) de taille égale à 70 µm de diamètre et ménagées sur tout ou partie de la longueur du tube, en particulier au niveau de la partie destinée à être en contact avec le tissu. Le tube est en outre obturé à son extrémité distale (29). Le diamètre extérieur du tube est égal à 200 µm, tandis que le diamètre intérieur est égal à 100 µm. De même que pour l'aiguille, l'extrémité amont du tube est munie d'un joint conique (30).

L'installation est mise en oeuvre de la manière suivante.

Dans un premier temps, on choisit une aiguille (23) ou un tube (27) présentant une répartition des perforations qui sont adaptées en fonction de la taille de la tumeur à traiter. Lorsque le moyen de diffusion (5) se présente sous forme d'un tube, celui-ci est positionné dans le tissu à traiter, avantageusement par l'intermédiaire d'une aiguille de ponction de diamètre supérieur servant de guide. Une fois positionné, l'extrémité libre du tube (27) communique avec l'extérieur de l'organisme. Le moyen de diffusion est alors connecté à la rallonge (3) par le biais du joint conique (30). L'extrémité libre de la rallonge (tube creux) (21) est alors connectée à l'ensemble unité de chauffage (3) / unité d'injection (1) par le biais du joint conique (22). Lorsque le moyen de diffusion se présente sous forme d'une aiguille, l'ensemble aiguille/rallonge soudé (26) est positionné au niveau de la tumeur à traiter.

L'opérateur détermine ensuite le volume d'eau à injecter en fonction de la taille de la tumeur. De par son expérience, le Demandeur a constaté qu'il était nécessaire, en général, d'injecter un volume de liquide représentant 5 % du volume de la tumeur à traiter pour obtenir une nécrose satisfaisante (à 400°C). L'opérateur détermine ensuite le volume de chaque injection et en déduit le nombre d'impulsions nécessaires pour parvenir à délivrer le volume total de liquide. Le déclenchement, la course, la force et la vitesse du vérin sont alors programmés pour permettre d'injecter N fois le volume de liquide à intervalles réguliers, en pratique compris entre 0,5 et 1 seconde par pulse de durée comprise entre 0,1 et 0,2 seconde. Dans un mode de réalisation avantageux, la dernière impulsion est d'une durée supérieure aux impulsions précédentes pour un même volume injecté, et ce, pour permettre de chauffer le moyen de diffusion avant son retrait, permettant ainsi d'éviter le risque de prolifération des cellules cancéreuses dans les tissus sains.

La manipulation débute alors en injectant le premier volume d'eau froide dans l'unité de chauffage. Ce volume est rapidement chauffé à 400°C, à une pression de 250 bars (1 seconde pour 1 ml avec 2 000 watts pour de l'eau). A l'impulsion d'eau froide suivante, l'eau chauffée est propulsée jusqu'au moyen de diffusion après ouverture de la soupape (7). L'eau pressurisée sort à l'extrémité du tube ou de l'aiguille à l'état de vapeur puis, la température diminuant par libération de calories dans la tumeur, la vapeur se transforme en eau chaude voisine de la température d'ébullition, c'est-à-dire 100°C, l'eau continuant alors à libérer les calories. L'injection de faibles volumes d'eau au niveau des perforations du moyen de diffusion entraîne un apport de calories localisé élevé, dont la diffusion se fait ensuite par conduction thermique.

Dans un mode de réalisation avantageux, on envoie un petit volume d'eau froide (2 à 5 % du volume d'eau chaude) dans le moyen d'amenée et le moyen de diffusion entre chaque injection d'eau chaude pressurisée, et ce, en programmant l'électrovanne (9). Cette variante permet d'éviter l'échauffement du moyen de diffusion, en particulier la partie non en contact avec les tissus sains entre chaque injection.

L'invention et les avantages qui en découlent ressortent bien de la description qui précède.

On notera en particulier l'aptitude de l'installation à assurer un régime d'administration de calories directement au sein d'un organisme, en régime pulsé, permettant ainsi de traiter des cellules cancéreuses et en particulier des tumeurs, par la chaleur, et ce, avec de très faibles volumes de liquide chauffé. En outre, la mise en oeuvre du faible volume d'eau permet d'éviter la diffusion de calories en dehors des tissus tumoraux et par ailleurs, le régime pulsé présente l'avantage de ne pas conduire à un échauffement non contrôlé du moyen de diffusion et partant une nécrose des tissus sains.

## Revendications

1. Installation destinée à la délivrance de calories au moyen d'un liquide, dans tout ou partie d'un tissu cellulaire humain ou animal comprenant :
- une unité de chauffage (3) dudit liquide,
- une unité d'injection (1) de liquide dans l'unité de chauffage,
- un moyen de diffusion (5) de liquide chauffé,
- un moyen d'amenée (4) du liquide chauffé depuis l'unité de chauffage jusqu'au moyen de diffusion,
**caractérisée en ce qu'**elle présente des moyens de délivrance du liquide chauffé au niveau du moyen de diffusion en régime pulsé.

2. Installation selon la revendication 1, **caractérisée en ce que** l'unité d'injection présente des moyens de délivrance du liquide chauffé à une pression au moins égale à la pression de vapeur saturante du liquide à injecter.

3. Installation selon l'une des revendications précédentes, **caractérisée en ce que** l'unité d'injection (1) se présente sous forme d'une chambre (10) contenant le liquide à injecter et dans laquelle un piston (12) est mû en translation sous l'action d'un vérin (11) électrique ou pneumatique, dont le déclenchement, la course, la force, et la vitesse de déplacement sont déterminés en fonction du rythme, du volume et de la pression d'injection souhaités du liquide, la chambre (10) communiquant avec l'unité de chauffage (3) par l'intermédiaire d'une première valve (6).

4. Installation selon la revendication 3, **caractérisée en ce que** le déclenchement et la course du vérin (11) sont programmés pour obtenir des volumes de liquide injectés compris entre 0,2 et 1 ml à intervalle régulier de 0,5 à 1 seconde.

5. Installation selon la revendication 4, **caractérisée en ce que** la force du vérin (11) est choisie de telle sorte à ce que le liquide soit injecté dans l'unité de chauffage (3) à une pression supérieure d'au moins 50 bars, avantageusement 100 bars à la pression de vapeur saturante dudit liquide.

6. Installation selon l'une des revendications précédentes, **caractérisée en ce qu'**elle comprend un réservoir de stockage de liquide (13) destiné à alimenter la chambre (10) de l'unité d'injection (1), ledit réservoir de stockage (13) étant séparé de la chambre (10) par une seconde valve (14).

7. Installation selon l'une des revendications précédentes, **caractérisée en ce que** l'unité de chauffage se présente sous forme d'une bobine métallique (15) incorporant une résistance électrique (17) ou un échangeur de température autour de laquelle est entouré un tube inox (16), dont le diamètre interne est compris entre 0,1 et 0,5 mm, la longueur du tube variant entre 1 500 et 5 000 mm.

8. Installation selon l'une des revendications précédentes, **caractérisée en ce que** l'unité de chauffage (3) est séparée du moyen d'amenée (4) par une soupape (7) tarée à une pression au moins égale à la pression de vapeur saturante du liquide à injecter.

9. Installation selon la revendication 8, **caractérisée en ce qu'**elle comprend un circuit de dérivation d'eau froide (8), dont le point de départ est positionné entre l'unité d'injection (1) et l'unité de chauffage (3) tandis que le point d'arrivée est positionnée en aval de la soupape (7), ledit circuit de dérivation (8) étant connecté au point de départ au moyen d'une électrovanne haute pression programmable (9).

10. Installation selon l'une des revendications précédentes, **caractérisée en ce que** le moyen d'amenée (4) se présente sous forme d'un tube creux (21) de diamètre intérieur compris entre 50 et 150 µm, la longueur du tube étant comprise entre 40 et 80 cm.

11. Installation selon l'une des revendications précédentes, **caractérisée en ce que** le moyen de diffusion (5) se présente sous forme d'un tube creux (27), dont l'extrémité distale (29) est obturée et dont les parois sont munies en tout ou partie de perforations (28) sensiblement circulaires de diamètre compris entre 30 et 70 µm, avantageusement 50 µm, le diamètre extérieur du tube étant compris entre 100 et 250 µm, tandis que le diamètre intérieur est compris entre 50 et 150 µm.

12. Installation selon l'une des revendications 1 à 10, **caractérisée en ce que** le moyen de diffusion se présente sous forme d'une aiguille rigide (23) dont l'extrémité distale (25) est obturée et dont les parois présentent, sur tout ou partie de leur longueur, des perforations (24) de diamètre compris entre 30 et 70 µm, avantageusement 50 µm, le diamètre intérieur de l'aiguille étant compris entre 0,05 et 0,2 mm, tandis que le diamètre extérieur est compris entre 0,2 mm et 0,7 mm, la longueur étant comprise entre 100 et 200 mm.

## Claims

1. An installation for delivering calories to all or part of human or animal cell tissue by means of a liquid, said installation comprising:
- a heating unit (3) for heating said liquid,
- an injection unit (1) for injecting liquid into the heating unit,
- a means (5) for diffusion of heated liquid,
- a means (4) for conveying the heated liquid from the heating unit to the diffusion means,
**characterized in that** it has means for delivering the heated liquid at the dispensing means in a pulsed operation.

2. The installation as claimed in claim 1, **characterized in that** the injection unit has means for delivering the heated liquid at a pressure at least equal to the saturation vapor pressure of the liquid to be injected.

3. The installation as claimed in one of the preceding claims, **characterized in that** the injection unit (1) is in the form of a chamber (10) which contains the liquid to be injected and in which a piston (12) is moved in translation under the action of an electric or pneumatic actuator (11) whose triggering, travel, force and speed of displacement are determined as a function of the desired rhythm, volume and pressure of injection of the liquid, the chamber (10) communicating with the heating unit (3) by way of a first valve (6).

4. The installation as claimed in claim 3, **characterized in that** the triggering and the travel of the actuator (11) are programmed in order to obtain injected volumes of liquid of between 0.2 and 1 ml at a regular interval of 0.5 to 1 second.

5. The installation as claimed in claim 4, **characterized in that** the force of the actuator (11) is chosen such that the liquid is injected into the heating unit (3) at a pressure greater by at least 50 bar, advantageously 100 bar, than the saturation vapor pressure of said liquid.

6. The installation as claimed in one of the preceding claims, **characterized in that** it comprises a liquid storage reservoir (13) intended to supply the chamber (10) of the injection unit (1), said storage reservoir (13) being separated from the chamber (10) by a second valve (14).

7. The installation as claimed in one of the preceding claims, **characterized in that** the heating unit is in the form of a metal coil (15) incorporating an electrical resistor (17) or a heat exchanger around which there is wound a stainless steel tube (16) whose internal diameter is between 0.1 and 0.5 mm, the length of the tube varying between 1500 and 5000 mm.

8. The installation as claimed in one of the preceding claims, **characterized in that** the heating unit (3) is separated from the conveying means (4) by a valve (7) calibrated to a pressure at least equal to the saturation vapor pressure of the liquid to be injected.

9. The installation as claimed in claim 8, **characterized in that** it comprises a cold water branch circuit (8) whose point of departure is positioned between the injection unit (1) and the heating unit (3), while the point of arrival is positioned downstream of the valve (7), said branch circuit (8) being connected at the point of departure by means of a programmable high-pressure solenoid valve (9).

10. The installation as claimed in one of the preceding claims, **characterized in that** the conveying means (4) is in the form of a hollow tube (21) with an internal diameter of between 50 and 150 µm, the length of the tube being between 40 and 80 cm.

11. The installation as claimed in one of the preceding claims, **characterized in that** the diffusion means (5) is in the form of a hollow tube (27) whose distal end (29) is closed off and whose walls are provided in whole or in part with substantially circular apertures (28) having a diameter of between 30 and 70 µm, advantageously 50 µm, the external diameter of the tube being between 100 and 250 µm, while the internal diameter is between 50 and 150 µm.

12. The installation as claimed in one of claims 1 through 10, **characterized in that** the diffusion means is in the form of a rigid needle (23) whose distal end (25) is closed off and whose walls have, along all or part of their length, apertures (24) with a diameter of between 30 and 70 µm, advantageously 50 µm, the internal diameter of the needle being between 0.05 and 0.2 mm, while the external diameter is between 0.2 mm and 0.7 mm, the length being between 100 and 200 mm.

## Patentansprüche

1. Vorrichtung, die mit Hilfe einer Flüssigkeit Wärmemengen in der Gesamtheit oder einem Teil eines menschlichen oder tierischen Zellgewebes freisetzen soll, umfassend:
- eine Aufheizeinheit (3) für die Flüssigkeit,
- eine Einheit zum Einspritzen (1) von Flüssigkeit in die Aufheizeinheit,
- ein Hilfsmittel für die Verteilung (5) der erhitzten Flüssigkeit,
- ein Hilfsmittel für die Zuführung (4) der erhitzten Flüssigkeit von der Aufheizeinheit zum Hilfsmittel für die Verteilung,
**dadurch gekennzeichnet, dass** sie Hilfsmittel zur Abgabe von erhitzter Flüssigkeit im Bereich des Verteilungshilfsmittels in gepulster Form aufweist.

2. Vorrichtung nach Anspruch 1, **dadurch gekennzeichnet, dass** die Einspritzeinheit Hilfsmittel für die Abgabe erhitzter Flüssigkeit mit einem Druck aufweist, der wenigstens gleich dem Sättigungsdampfdruck der einzuspritzenden Flüssigkeit ist.

3. Vorrichtung nach einem der vorstehenden Ansprüche, **dadurch gekennzeichnet, dass** die Einspritzeinheit (1) in Form einer Kammer (10) vorliegt, die die einzuspritzende Flüssigkeit enthält und in der ein Kolben (12) unter der Wirkung eines elektrischen Zylinders oder Druckzylinders (11) in Translationsbewegung versetzt wird, dessen Auslösen, Hub, Kraft und Versetzungsgeschwindigkeit durch den erwünschten Rhythmus, das erwünschte Volumen und den erwünschten Einspritzdruck der Flüssigkeit bestimmt sind, wobei die Kammer (10) über ein erstes Ventil (6) mit der Aufheizeinheit (3) verbunden ist.

4. Vorrichtung nach Anspruch 3, **dadurch gekennzeichnet, dass** das Auslösen und der Hub des Zylinders (11) programmiert sind, um Volumina eingespritzter Flüssigkeit zwischen 0,2 und 1 ml in einem regelmäßigen Intervall von 0,5 bis 1 Sekunde zu erhalten.

5. Vorrichtung nach Anspruch 4, **dadurch gekennzeichnet, dass** die Kraft des Zylinders (11) so ausgewählt ist, dass die Flüssigkeit mit einem Druck in die Aufheizeinheit (3) eingespritzt wird, der wenigstens 50 bar, vorteilhaft 100 bar höher ist als der Sättigungsdampfdruck der Flüssigkeit.

6. Vorrichtung nach einem der vorstehenden Ansprüche, **dadurch gekennzeichnet, dass** sie einen Vorratsbehälter für Flüssigkeit (13) umfasst, der die Kammer (10) der Einspritzeinheit (1) versorgen soll, wobei der Vorratsbehälter (13) durch ein zweites Ventil (14) von der Kammer (10) getrennt ist.

7. Vorrichtung nach einem der vorstehenden Ansprüche, **dadurch gekennzeichnet, dass** die Aufheizeinheit in Form einer metallischen Spule (15) vorliegt, die einen elektrischen Widerstand (17) oder einen Wärmetauscher enthält und von einem Edelstahltubus (16) umgeben ist, dessen Innendurchmesser zwischen 0,1 und 0,5 mm beträgt, wobei die Länge des Tubus zwischen 1500 und 5000 mm variiert.

8. Vorrichtung nach einem der vorstehenden Ansprüche, **dadurch gekennzeichnet, dass** die Aufheizeinheit (3) von dem Zuführungshilfsmittel (4) durch ein Ventil (7) getrennt ist, das auf einen Druck geeicht ist, der wenigstens gleich dem Sättigungsdampfdruck der einzuspritzenden Flüssigkeit ist.

9. Vorrichtung nach Anspruch 8, **dadurch gekennzeichnet, dass** sie einen Nebenkreislauf mit kaltem Wasser (8) umfasst, dessen Ausgangspunkt zwischen der Einspritzeinheit (1) und der Aufheizeinheit (3) angeordnet ist, während der Zuführungspunkt unterhalb des Ventils (7) angeordnet ist, wobei der Nebenkreislauf (8) mit Hilfe eines programmierbaren Hochdruck-Elektroventils (9) mit dem Ausgangspunkt verbunden ist.

10. Vorrichtung nach einem der vorstehenden Ansprüche, **dadurch gekennzeichnet, dass** das Zuführungshilfsmittel (4) in Form eines hohlen Tubus (21) vorliegt, der einen Innendurchmesser zwischen 50 und 150 µm hat, wobei die Länge des Tubus zwischen 40 und 80 cm liegt.

11. Vorrichtung nach einem der vorstehenden Ansprüche, **dadurch gekennzeichnet, dass** das Verteilungshilfsmittel (5) in Form eines hohlen Tubus (27) vorliegt, dessen distales Ende (29) verschlossen ist und dessen Wände vollständig oder teilweise mit in etwa kreisförmigen Löchern (28) eines Durchmessers zwischen 30 und 70 µm, vorteilhaft 50 µm, versehen sind, wobei der Außendurchmesser des Tubus zwischen 100 und 250 µm beträgt, während der Innendurchmesser zwischen 50 und 150 µm beträgt.

12. Vorrichtung nach einem der Ansprüche 1 bis 10, **dadurch gekennzeichnet, dass** das Verteilungshilfsmittel in Form einer steifen Nadel (23) vorliegt, deren distales Ende (25) verschlossen ist und deren Wände auf ihrer gesamten Länge oder einem Teil derselben Löcher (24) eines Durchmessers zwischen 30 und 70 µm, vorzugsweise 50 µm aufweisen, wobei der Innendurchmesser der Nadel zwischen 0,05 und 0,2 mm beträgt, während der Außendurchmesser zwischen 0,2 mm und 0,7 mm beträgt und die Länge derselben zwischen 100 und 200 mm liegt.
